# EUROPEAN PATENT APPLICATION

(11) **EP 4 368 112 A1**
(43) Date of publication of application: **15.05.2024**
(21) Application number: 23208182.8
(22) Date of filing: 07.11.2023
(51) Int. Cl.: A61B 6/46, A61B 8/00, G06T 7/00

(54) **IMAGE PROCESSING APPARATUS AND IMAGE PROCESSING METHOD**

(30) Priority: 11.11.2022 JP 2022180791
(71) Applicant: CANON KABUSHIKI KAISHA, Tokyo 146-8501 (JP)
(72) Inventor: OTOMARU, Itaru, Tokyo (JP)
(74) Representative: WESER & Kollegen Patentanwälte PartmbB

(57) **Abstract**

An image processing apparatus includes at least one memory and at least one processor which function as an image acquiring unit configured to reduce a resolution of the three-dimensional image and acquire a three-dimensional image of an object, an initial parameter estimating unit configured to estimate an initial parameter in accordance with an initial value of an adjustable parameter to be utilized in obtaining a reference cross section, a cross sectional image acquiring unit configured to acquire one or more cross sectional images based on the three-dimensional image and the initial parameter, and an indicator estimating unit configured to estimate an indicator o be contributed to correcting the initial parameter, wherein the estimated indicator is in association with a specified cross sectional image out of the one or more cross sectional images.

## Description

### BACKGROUND OF THE INVENTION

### Field of the Invention

The technique according to the present disclosure relates to an image processing apparatus and an image processing method.

### Description of the Related Art

In diagnoses using medical images, a diagnosis may be performed by cutting a three-dimensional image (volume data) based on a prescribed criterion and displaying a two-dimensional cross section (reference cross section) being a cut surface on a display or the like. However, since manually setting a reference cross section is accompanied by an operation to find an anatomical landmark (feature point) or the like which provides a clue in a three-dimensional space, there is a problem in that a large burden is placed on a physician or the like.

Various techniques for automatically estimating a reference cross section have been proposed to solve this problem. Japanese Patent Application Laid-open No. 2011-161213 discloses a method of estimating a reference cross section of a three-dimensional moving image made up of a plurality of time phases at which a heart region has been imaged. In Japanese Patent Application Laid-open No. 2011-161213, a reference cross section is estimated at a specific reference time phase among the plurality of time phases and, by moving and rotating the reference cross section by tracking between time phases, a reference cross section of a time phase other than the reference time phase is obtained.

In addition, in WO 2016/195110, a candidate of a reference cross section (candidate cross section) is obtained from an image obtained by capturing a heart region and feature point coordinates are calculated from each of a plurality of cross sections obtained by translating and rotating the candidate cross section. The feature points are integrated to correct the candidate cross section.

### SUMMARY OF THE INVENTION

However, with reference cross section estimation using the methods described in the conventional art described above, an inference with sufficient accuracy is not always drawn.

The technique according to the present disclosure has been devised in consideration thereof and provides a technique that enables estimation accuracy of a reference cross section of a three-dimensional image to be improved.

According to some embodiments, there is provided an image processing apparatus as specified in claims 1 to 20.

Furthermore, the technique according to the present disclosure can also be considered an image processing method configured to include at least a part of the processing described above, a program causing a computer to execute the methods, or a computer-readable recording medium which records such a program on a non-transitory basis. The respective components and processing described above can be implemented in combination with one another in any way possible insofar as technical contradictions do not arise.

Further features of the present invention will become apparent from the following description of exemplary embodiments with reference to the attached drawings.

### BRIEF DESCRIPTION OF THE DRAWINGS

FIG. 1 is a diagram showing a schematic configuration of an image processing apparatus according to an embodiment;
FIG. 2 is a flow chart of processing executed by the image processing apparatus according to the embodiment;
FIGS. 3A to 3C are diagrams showing a clinical definition of a reference cross section according to the embodiment;
FIGS. 4A to 4F are diagrams schematically showing a reference cross section calculated by the image processing apparatus according to the embodiment;
FIGS. 5A to 5C are diagrams schematically showing estimation processing of a correction parameter by the image processing apparatus according to the embodiment;
FIG. 6 is a flow chart of processing executed by an image processing apparatus according to an embodiment; and
FIG. 7 is another flow chart of processing executed by an image processing apparatus according to an embodiment.

### DESCRIPTION OF THE EMBODIMENTS

Hereinafter, embodiments of the present disclosure will be described with reference to the drawings. It is to be understood that the present disclosure is not limited to the embodiments described below and modifications may be appropriately made without departing from the spirit and scope of the disclosure. In addition, elements with the same function in the drawings described below may be denoted by same reference characters and a description thereof may be either omitted or simplified.

The image processing apparatuses according to the embodiments described below provide a function of estimating a prescribed reference cross section which is to be used by a physician or the like to observe (diagnosis) an input three-dimensional image. An input image to be a processing object is a medical image or, in other words, an image of an object (such as the human body) photographed or generated for the purpose of carrying out a medical diagnosis, examination, research, and the like and is, typically, an image acquired by an image capturing system called a modality. Examples of the input image include an ultrasonic image that is obtained by an ultrasonic diagnostic apparatus, an X-ray CT image that is obtained by an X-ray CT apparatus, and an MRI image that is obtained by an MRI apparatus.

Hereinafter, a specific example of an image processing apparatus will be described in detail by citing, as an example, a case where a transesophageal three-dimensional ultrasonic image obtained by capturing a mitral valve region of the heart is an input three-dimensional image and a position of an anatomical landmark in the mitral valve region being an observation object is to be estimated. Note that making the mitral valve an observation object is merely an example and another site may be made an observation object.

### First Embodiment

The image processing apparatus according to the first embodiment uses a three-dimensional image as an input image to estimate a parameter (reference cross section parameter) representing a position and an attitude of a two-dimensional reference cross section. In estimation processing, first, a reference cross section parameter is estimated on the basis of the input image. Hereinafter, the parameter that is estimated at this stage will be referred to as an "initial value of the reference cross section parameter" (initial parameter) as an initial value of a parameter for obtaining a reference cross section. Next, based on the initial parameter, three cross sectional images named an A plane, a B plane, and a C plane are segmented from the input image. In addition, a correction amount of a respectively different component in the reference cross section parameter is estimated from each cross sectional image. In the following description, the correction amount will be referred to as a "correction amount of the reference cross section parameter" (or, simply, a "correction amount"). Finally, the correction amount estimated for each cross sectional image is applied to the initial parameter to obtain a final reference cross section parameter. Hereinafter, the parameter that is finally obtained in this manner will be referred to as a "final value of the reference cross section parameter" (final parameter).

As described above, the reference cross section parameter is a parameter that represents a position and an attitude of a reference cross section. In addition, the reference cross section parameter in the present example is synonymous with a parameter that represents a position and an attitude in the input three-dimensional image of a coordinate system (observation object coordinate system) that represents an observation object (in the present example, the mitral valve). In the present example, it is assumed that the position is expressed by three parameters representing three-dimensional coordinates (cx, cy, cz) of a reference position (hereinafter, also referred to as a center position) of the mitral valve to be described later. In addition, it is assumed that the attitude is expressed by a total of six parameters including three parameters representing a long axis vector (lx, ly, lz) and a short axis vector (sx, sy, sz) which are defined with respect to the mitral valve to be described later. Note that the reference cross section parameter described above is merely exemplified as an embodiment of the technique according to the present disclosure and any expression form may be adopted as long as the information represents the position and attitude of the reference cross section. Details of initial parameter estimation processing of the reference cross section will be described in the description of step S203. In the present embodiment, it is assumed that a position and an attitude of each cross section of the A plane, the B plane, and the C plane in the observation object coordinate system are known. Therefore, the position and the attitude of each cross section of the A plane, the B plane, and the C plane in the input three-dimensional image are uniquely calculated from the reference cross section parameter that is expressed by the nine parameters described above. Therefore, a cross sectional image acquired by a cross sectional image acquiring unit 43 is constituted of a plurality of images of which positions and attitudes among the images have a prescribed relationship.

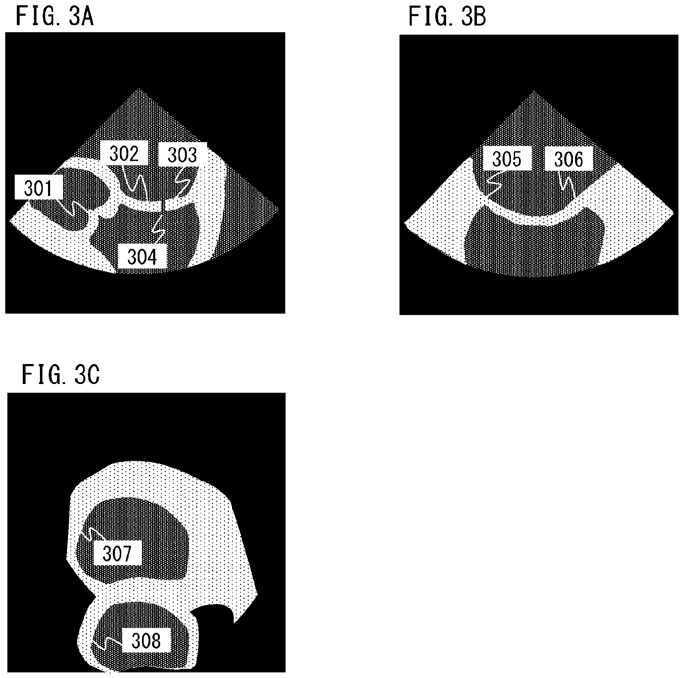

FIGS. 3A to 3C schematically show a clinical definition of each cross section. FIG. 3A represents an example of the A plane and shows a cross section that enables the aortic valve (301), the anterior cusp (302) of the mitral valve, and the posterior cusp (303) of the mitral valve to be simultaneously observed. FIG. 3B represents an example of the B plane and shows a cross section which intersects the A plane at a boundary (304) between the anterior cusp and the posterior cusp and which captures the mitral annulus (305 and 306) that is centered on the boundary. FIG. 3C represents an example of the C plane and shows a cross section which slices the mitral valve (307) and the aortic valve (308).

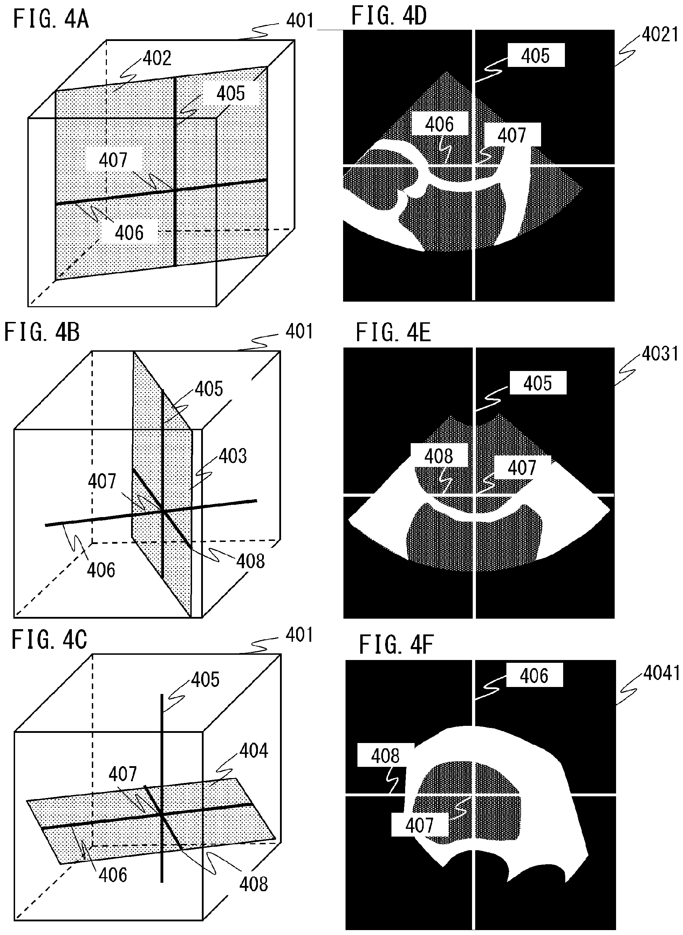

Based on the clinical definitions shown in FIGS. 3A to 3C, a definition of each cross section in the present embodiment is shown in FIGS. 4A to 4E. FIGS. 4A to 4C show relationships of positions and attitudes of the A plane (402), the B plane (403), and the C plane (404) with respect to an input three-dimensional image (401). The A plane, the B plane, and the C plane intersect at one point that is a center position (407) and are orthogonal to each other. As shown in FIG. 4D, a long axis (405) is an axis that penetrates the mitral valve in an up-down direction and constitutes an in-plane vector in the A plane. A short axis (406) that is orthogonal to the long axis is an axis that slices the mitral valve at the heights of the anterior cusp and the posterior cusp and the axis 405 and the axis 406 intersect each other at the point 407. The B plane is a cross section created by rotating the A plane around the long axis (405) by 90 degrees. The C plane is a cross section that is orthogonal to both the A plane and the B plane and the axis 406 and an axis 408 that indicate in-plane vectors of the C plane coincide with in-plane vectors that respectively penetrate the A plane and the B plane in a left-right direction.

As described above, a calculation of a correction amount of the reference cross section parameter is performed by calculating a correction amount of a different component of the reference cross section parameter for each cross sectional image. In the A plane, an in-plane component of the A plane at the center position (cx, cy, cz) or, in other words, by how much the center position is to be translated in-plane of the A plane is calculated. In the B plane, an in-plane component of the long axis vector or, in other words, by how much an axis that penetrates the B plane in the up-down direction in the initial parameter is to be rotated in the B plane is calculated. In addition, in the C plane, an in-plane component of the short axis vector or, in other words, by how much an axis that penetrates the C plane in the up-down direction in the initial parameter is to be rotated in the C plane is calculated. Details of the calculation processing of the correction amounts will be described in the description of step S205. In addition, details of processing of reflecting the correction amounts onto the initial parameter and obtaining the final parameter will be described in the description of step S206.

Note that a form other than that described above may be used as an expression form of the reference cross section parameter as long as the positions and the attitudes of three cross sections that are orthogonal to each other in a three-dimensional space can be expressed. For example, an attitude may be expressed with four parameters (three parameters of a rotation axis vector and one parameter of a rotation angle) using Rodrigues' rotation formula which is a known technique. In this case, the reference cross section parameter is to have a total of seven parameters including the three parameters of the center position. Alternatively, the position and the attitude can be expressed by a four by four rigid transformation matrix. Since these expression forms are mutually convertible, processing according to the present embodiment can be executed in any case.

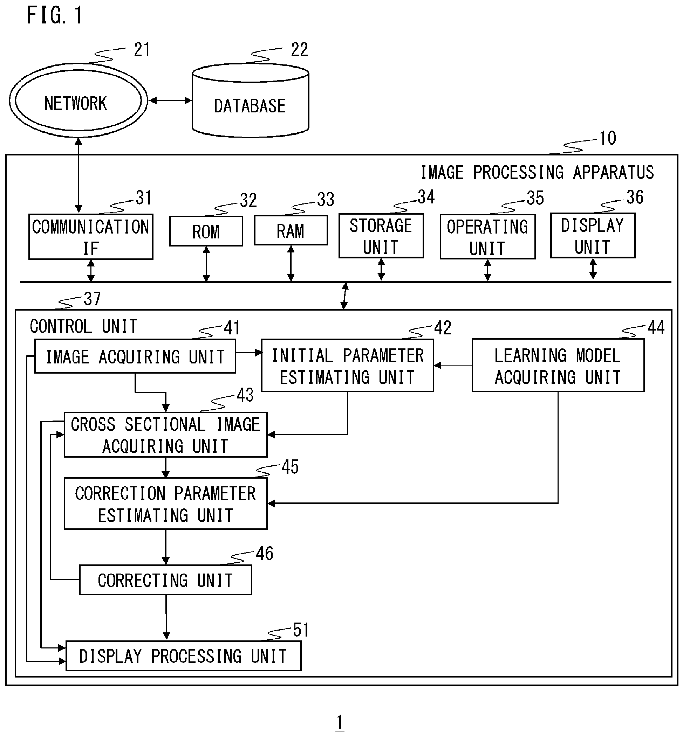

Hereinafter, a configuration and processing of the image processing apparatus according to the present embodiment will be described. FIG. 1 is a block diagram showing a configuration example of an image processing system (also referred to as a medical image processing system) which includes the image processing apparatus according to the present embodiment. An image processing system 1 includes an image processing apparatus 10 and a database 22. The image processing apparatus 10 is connected to the database 22 via a network 21 in a state where the image processing apparatus 10 can communicate with the database 22. For example, the network 21 includes a LAN (Local Area Network) or a WAN (Wide Area Network).

The database 22 stores and manages a plurality of images and information to be used in processing to be described below. The information managed in the database 22 includes an input three-dimensional image to be an object of reference cross section estimation processing by the image processing apparatus 10 and information on a learning model to be used in each step of estimation processing. The image processing apparatus 10 is capable of acquiring data stored in the database 22 via the network 21. Note that the information on the learning model may be stored in an internal storage (a ROM 32 or a storage unit 34) of the image processing apparatus 10 instead of the database 22.

The image processing apparatus 10 includes a communication IF (interface) 31, the ROM (Read Only Memory) 32, a RAM (Random Access Memory) 33, the storage unit 34, an operating unit 35, a display unit 36, and a control unit 37.

The communication IF 31 is a communicating unit which is constituted of a LAN card or the like and which realizes communication between an external apparatus (for example, the database 22) and the image processing apparatus 10. The ROM 32 is constituted of a nonvolatile memory or the like and stores various program and various data. The RAM 33 is constituted of a volatile memory or the like and is used as a work memory that temporarily stores a program being executed and data. The storage unit 34 is constituted of an HDD (a hard disk drive) or the like and stores various program and various data. The operating unit 35 is constituted of a keyboard, a mouse, a touch panel, or the like and inputs an instruction from a user (for example, a physician or a medical technologist) to various apparatuses. The display unit 36 is constituted of a display or the like and displays various kinds of information to the user.

The control unit 37 is constituted of a CPU (Central Processing Unit) or the like and controls processing in the image processing apparatus 10 in an integrated manner. As functional components, the control unit 37 includes an image acquiring unit 41, an initial parameter estimating unit 42, the cross sectional image acquiring unit 43, a learning model acquiring unit 44, a correction parameter estimating unit 45, a correcting unit 46, and a display processing unit 51. The control unit 37 may include a GPU (Graphics Processing Unit), a DSP (Digital Signal Processor), or an FPGA (Field Programmable Gate Array).

The image acquiring unit 41 acquires, from the database 22, an input three-dimensional image that is a three-dimensional image of an object to be input to the image processing apparatus 10. The input three-dimensional image may be directly acquired from the modality. In such a case, the image processing apparatus 10 may be implemented inside of a console of the modality (image capturing system). Details of the processing will be described in the description of step S201.

The initial parameter estimating unit 42 estimates an initial parameter that is an initial value of a parameter for reducing a resolution of the input three-dimensional image acquired by the image acquiring unit 41 to obtain a reference cross section. Details of the processing will be described in the description of step S203.

The learning model acquiring unit 44 acquires a learning model to be used by the initial parameter estimating unit 42 and the correction parameter estimating unit 45 from the database 22. Details of the processing will be described in the description of step S202.

The cross sectional image acquiring unit 43 uses the input three-dimensional image acquired by the image acquiring unit 41 and the initial parameter of the reference cross section estimated by the initial parameter estimating unit 42 to acquire at least one two-dimensional cross sectional image from the three-dimensional image. In addition, the cross sectional image acquiring unit 43 uses the final parameter corrected by the correcting unit 46 to acquire at least one two-dimensional cross sectional image from the three-dimensional image. Details of the processing will be described in the description of steps S204 and S207.

The correction parameter estimating unit 45 is an indicator estimating unit which uses each cross sectional image acquired by the cross sectional image acquiring unit 43 to estimate a correction amount of the reference cross section parameter for each cross sectional image. In this case, the correction amount of the reference cross section parameter is an example of an indicator which contributes to correction of the initial parameter.
A correction amount of the center position is estimated from the A plane and correction amounts of orientations of axes (respectively referred to as a long axis and a short axis) which penetrate respective cross sections in the up-down direction are estimated from the B plane and the C plane. Details of the processing will be described in the description of step S205.

The correcting unit 46 calculates a final parameter by reflecting the correction amounts of the reference cross section parameter estimated by the correction parameter estimating unit 45 onto the initial parameter estimated by the initial parameter estimating unit 42. Details of the processing will be described in the description of step S206.

The display processing unit 51 displays, in an image display region of the display unit 36, a cross sectional image obtained by cutting the input three-dimensional image based on the reference cross section parameter calculated by the correcting unit 46 in a display mode that enables the user of the image processing apparatus 10 to visibly check the cross sectional image. Details of the processing will be described in the description of step S208.

Each constituent element of the image processing apparatus 10 described above functions according to a computer program. For example, the function of each constituent element is realized as the control unit 37 (CPU) uses the RAM 33 as a work area to read a computer program stored in the ROM 32, the storage unit 34, or the like and executes the computer program. Note that a part of or all of the functions of the constituent elements of the image processing apparatus 10 may be realized using a dedicated circuit. In addition, a part of the functions of the constituent elements of the control unit 37 may be realized using a cloud computer. For example, an arithmetic processing device at a location that differs from the image processing apparatus 10 may be connected to the image processing apparatus 10 via the network 21 so as to be capable of communicating with the image processing apparatus 10. In addition, the functions of the constituent elements of the image processing apparatus 10 or the control unit 37 may be realized as the image processing apparatus 10 performs transmission/reception of data to/from the arithmetic processing device.

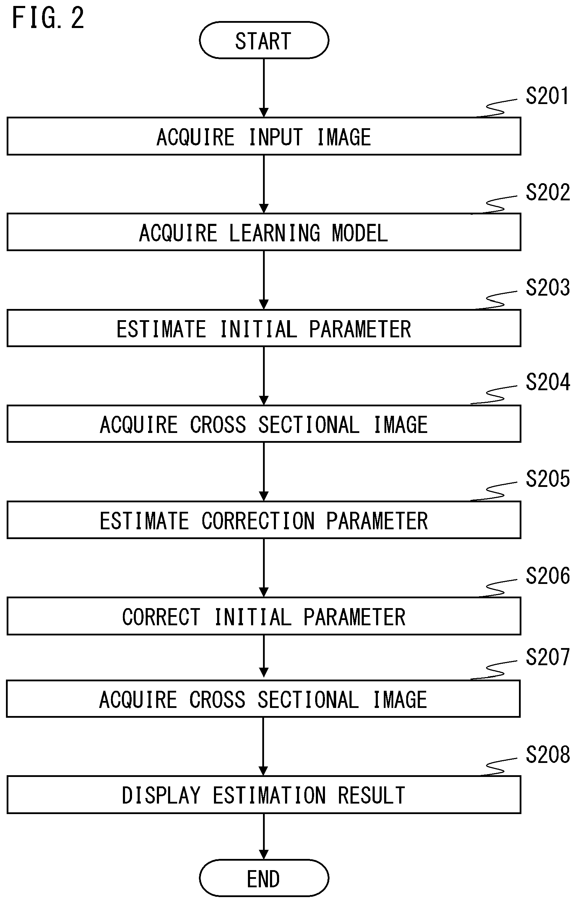

Next, an example of processing of the image processing apparatus 10 shown in FIG. 1 will be described with reference to the flow chart shown in FIG. 2.

(Step S201: Acquisition of Input Image) In step S201, the image processing apparatus 10 acquires an instruction of image acquisition from the user via the operating unit 35. In addition, the image acquiring unit 41 acquires the input three-dimensional image designated by the user from the database 22 and stores the input three-dimensional image in the RAM 33. Note that besides acquiring the input three-dimensional image from the database 22, an input image may be acquired from among ultrasonic images constantly being captured by an ultrasonic diagnostic apparatus.

(Step S202: Acquisition of Learning Model) In step S202, the learning model acquiring unit 44 acquires, from the database 22, two types of learning models to be respectively used by the initial parameter estimating unit 42 and the correction parameter estimating unit 45 to perform estimation and stores the learning models in the RAM 33.

The learning model to be used by the initial parameter estimating unit 42 is configured to express a statistical trend of a relationship between the input three-dimensional image and the reference cross section parameter that is an output value. Therefore, the learning model acquired by the initial parameter estimating unit 42 is a learning model for initial parameter estimation created by learning processing using information including a set of a three-dimensional image for learning and a parameter for obtaining a reference cross section in the three-dimensional image for learning.

On the other hand, the learning model to be used by the correction parameter estimating unit 45 differs among the respective cross sectional images (A plane, B plane, and C plane) and expresses a statistical trend of a relationship between an input cross sectional image and a correction amount of the reference cross section parameter in the cross section. In other words, the following three learning models are acquired: a learning model for estimating a correction amount of an in-plane component of the center position from an image of the A plane; a learning model for estimating a correction amount of an in-plane angle of the long axis vector from an image of the B plane; and a learning model for estimating a correction amount of an in-plane angle of the short axis vector from an image of the C plane. Therefore, the learning model acquired by the correction parameter estimating unit 45 is a learning model for correction parameter estimation created by learning processing using information including a set of a cross sectional image for learning and an indicator that contributes toward correcting the reference cross section parameter in the cross sectional image for learning. Details of the correction parameter estimation processing in each cross sectional image will be described in the description of step S205.

In the present embodiment, a convolutional neural network (CNN) is used as a learning model. In this case, by performing learning using a large number of pieces of case data in which an input and an output form a set as learning data, a learning model to perform each estimation is to be constructed in advance. In the present processing step, learning models constructed in this manner are acquired. Note that besides acquiring learning models constructed in advance from the database 22, a processing unit that performs the learning processing described above may be constructed in the control unit of the image processing apparatus 10 and learning of the present step may be performed by the processing unit.

(Step S203: Estimation of Initial Parameter) In step S203, the initial parameter estimating unit 42 inputs volume data of the input three-dimensional image and outputs an initial parameter of a reference cross section made up of a center position, a long axis vector, and a short axis vector.

As described in step S202, in the present embodiment, estimation of the initial parameter is performed using a convolutional neural network (CNN) that estimates the initial parameter from the input three-dimensional image. In other words, a reference cross section parameter made up of nine parameters which is obtained as an output of a CNN when a three-dimensional image is input to the CNN is acquired as the initial parameter.

In this case, the three-dimensional image that is input to the CNN is not the same as the input three-dimensional image acquired in step S201 and is a "rough" image with reduced resolution. For example, let us assume that the input three-dimensional image is a volume image expressing a range of 256 × 256 × 256 voxels when a length per voxel is 0.6 mm or, in other words, a range of 153.6 mm per side. In the present step, the number of voxels per side of the input three-dimensional image is reduced to 1/8 to 32 × 32 × 32 voxels. In other words, while the expressed range remains unchanged at 153.6 mm per side, conversion is performed to an image of which a length per voxel is 4.8 mm. Accordingly, compared to a case where the input three-dimensional image is used as-is, a calculation time and a used memory amount which are necessary for inference using a CNN can be reduced. In addition, known image processing such as pixel value normalization or contrast correction using a mean or a dispersion of pixel values of the input three-dimensional image can be further applied. Any known method may be used in the resolution conversion processing described above. For example, voxel values may be sampled in steps according to a diminution or a mean value of pixel values of voxels in a range according to the diminution can be used. In addition, the resolution conversion processing and image processing such as pixel value normalization described above can be executed in any order.

While the present embodiment assumes that a reference cross section is to be expressed by nine parameters and the processing of the present step is described using a case of a direct estimation using the nine values as an output of estimation by a CNN as an example, other expression forms may be used as the output of the estimation by a CNN. For example, the reference cross section parameter may be expressed by seven parameters (three parameters of the center position, three parameters of the rotation axis vector, and one parameter of the rotation angle) and the estimation by a CNN may output the seven parameters. In this case, a conversion to the expression form of the nine parameters described above may be performed after the estimation by a CNN. In a similar manner, the estimation by a CNN may output a four by four rigid transformation matrix.

While a case where the A plane, the B plane, and the C plane are orthogonal to each other will be described as an example in the present embodiment, the A plane, the B plane, and the C plane need not necessarily be orthogonal to each other as long as a relationship among the positions and the attitudes of the respective cross sections is defined in advance. For example, the B plane may be defined as a cross section which is not orthogonal to the A plane and which is obtained by rotating the A plane around the long axis by 80 degrees. Even in this case, the processing of the present step can be performed in a similar manner to a case where the three cross sections are orthogonal to each other. In addition, if the position and the attitude of each cross section in the observation object coordinate system are known, an intersecting position of the three cross sections need not necessarily coincide with the center position of the reference cross section parameter (= reference position of the observation object). In a similar manner, the three cross sections may not be configured to respectively share in-plane axes and an image center of each reference cross section image need not necessarily coincide with the center position of the reference cross section parameter or the intersecting position of the three cross sections.

(Step S204: Acquisition of Sectional Image) In step S204, the cross sectional image acquiring unit 43 segments a two-dimensional cross sectional image using the input three-dimensional image and the initial parameter estimated in step S203. Note that in the present embodiment, each two-dimensional cross sectional image is acquired by segmenting the cross sectional image so that the center position of the initial parameter of the reference cross section coincides with a center of the cross sectional image. In other words, even when the center position (407) is not the center of the input three-dimensional image (401) as shown in FIGS. 4A to 4C, a center of each cross sectional image of the A plane (4021), the B plane (4031), and the C plane (4041) is the center position (407) as shown in FIGS. 4D to 4F.

In this case, a spatial resolution of the two-dimensional cross sectional image is assumed to be higher than a resolution of the three-dimensional image after reducing the resolution used in step S203. For example, it is assumed that a 128 × 128 pixel image with a per-pixel length (pixel size) of 1.2 mm is used as the two-dimensional cross sectional image. In this case, an image with four times the resolution per side as compared to the image with a per-voxel length of 4.8 mm used in step S203 is obtained. Accordingly, an image with a fine resolution can be used in reference cross section parameter estimation without significantly increasing a calculation amount and a used memory amount.

The cross sectional image acquisition processing will now be described in greater detail with reference to FIGS. 4A to 4F. When acquiring the A plane cross sectional image (4021), first, the input three-dimensional image (401) is sampled with 407 as the center position, the axis 405 as the up-down direction vector, and the axis 406 as the left-right direction vector. In this case, it is assumed that the per-pixel length is the same (0.6 mm) as the input three-dimensional image and that a range of 256 × 256 pixels (153.6 mm × 153.6 mm) is to be sampled. At this point, when a sampling position exceeds a defined range of the input three-dimensional image, a pixel value of the position is assumed to be 0. Next, a resolution of the two-dimensional image of 256 × 256 pixels obtained in this manner is reduced by 1/2 to generate a two-dimensional image of 128 × 128 pixels. In other words, while the defined range remains unchanged at 153.6 mm × 153.6 mm, a two-dimensional image with a per pixel length of 1.2 mm is obtained. Accordingly, the A plane cross sectional image can be obtained. The B plane cross sectional image (4031) is calculated by the same procedure as the A plane cross sectional image with the axis 405 as the up-down direction vector and the axis 408 as the left-right direction vector. The C plane cross sectional image (4041) is also calculated by the same procedure as the A plane and B plane cross sectional images with the exception of using the axis 406 as the up-down direction vector and the axis 408 as the left-right direction vector.

(Step S205: Estimation of Correction Parameter) In step S205, with each cross sectional image calculated in step S204 as an input, the correction parameter estimating unit 45 uses a learning model to calculate a correction amount of a component of the reference cross section parameter corresponding to each type of cross section. Accordingly, the correction parameter estimating unit 45 estimates an indicator that contributes toward correction of the initial parameter in an image plane of a cross sectional image. In this case, correction amount estimation with respect to the A plane cross sectional image, the B plane cross sectional image, and the C plane cross sectional image is to be respectively independently executed. The estimation of any of the cross sectional images is to be performed using a CNN to which each cross sectional image is input.

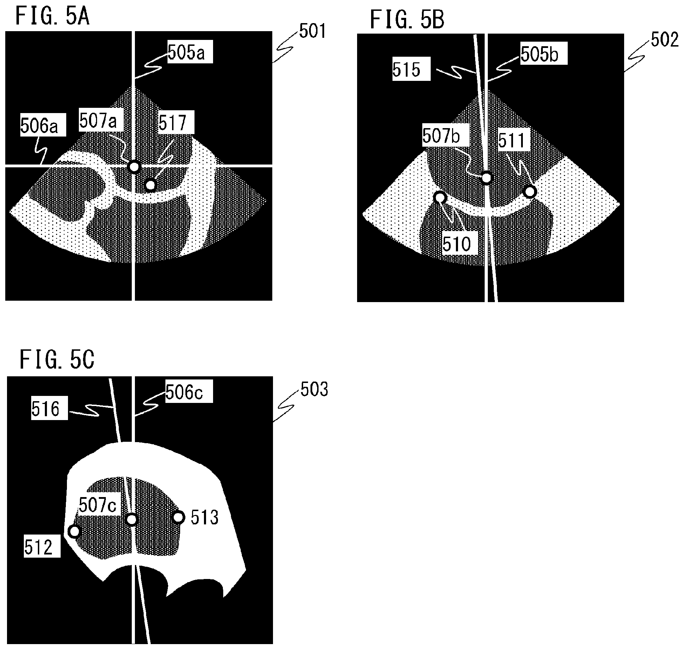

Estimation processing of a correction parameter that is a correction amount for moving the reference cross section obtained by the initial parameter will now be described in specific terms. Estimation processing of the correction parameter using an A plane cross sectional image will be described with reference to FIG. 5A. In the A plane cross sectional image (501) acquired based on the initial parameter, a center position (507a) of the reference cross section is positioned at the center of the A plane cross sectional image. In this case, in the present step, an amount of displacement for moving the position of the reference cross section obtained by the initial parameter to a target position of the reference cross section which indicates "by how much the center position is to be moved in the A plane" is estimated. In other words, how much the center position having been estimated as the initial parameter in step S203 has deviated from a center position to be a target position of a reference cross section that is determined by a prescribed criterion (for example, an anatomical meaning or a clinical guideline) is estimated. Instead of estimating the movement amount of the center position, a center position after correction (a two-dimensional coordinate value in the A plane) may be estimated. In this case, for example, as indicated by a point 517 in FIG. 5A, a position that differs from the original center position is estimated as the center position after the correction.

Estimation processing of the correction parameter using a B plane cross sectional image will be described with reference to FIG. 5B. In the initial parameter, a long axis vector (505b) is an axis that penetrates the B plane cross sectional image in the up-down direction. In this case, in the present step, an amount of rotation for rotating an attitude of a reference cross section obtained by the initial parameter to a target attitude of the reference cross section which indicates "by how much the long axis vector is to be rotated in the B plane" is estimated. In other words, how much the long axis vector having been estimated as the initial parameter in step S203 has deviated from a long axis vector to be a target of a reference cross section that is determined by a prescribed criterion (an anatomical meaning or a clinical guideline) is estimated. Instead of estimating the rotation amount of the long axis vector, an orientation of the long axis vector after correction (a two-dimensional vector in the B plane) may be estimated. In this case, for example, as indicated by a vector 515 in FIG. 5B, a vector with an orientation that differs from that of the original long axis vector is estimated as the long axis vector after the correction.

Alternatively, instead of directly estimating the long axis vector after correction, the long axis vector after correction may be indirectly calculated from an estimation result of another indicator. For example, a coordinate value of two points (points 510 and 511 in FIG. 5B) at which the B plane intersects with the mitral annulus may be estimated and a vector that is orthogonal to a vector connecting the two points may be calculated as the long axis vector after correction. Alternatively, a coordinate value of two points (not illustrated) which differ from the two points and through which a long axis is to pass may be estimated and a vector connecting the two estimated points may be calculated as the long axis vector after correction.

Estimation processing of the correction parameter using a C plane cross sectional image will be described with reference to FIG. 5C. In the initial parameter, a short axis vector (506c) is an axis that penetrates the C plane cross sectional image in the up-down direction. In this case, in the present step, an amount of rotation for rotating an attitude of a reference cross section obtained by the initial parameter to a target attitude of the reference cross section which indicates "by how much the short axis vector is to be rotated in the C plane" is estimated. In other words, how much the short axis vector having been estimated as the initial parameter in step S203 has deviated from a short axis vector of a target of a reference cross section that is determined by a prescribed criterion (an anatomical meaning or a clinical guideline) is estimated.

In a similar manner to the B plane, instead of estimating the rotation amount of the short axis vector, an orientation of the short axis vector after correction (a two-dimensional vector in the C plane) may be estimated. In this case, for example, as indicated by a vector 516 in FIG. 5C, a vector with an orientation that differs from that of the original short axis vector is estimated as the short axis vector after the correction.

Alternatively, instead of directly estimating the short axis vector after correction, the short axis vector after correction may be indirectly calculated from an estimation result of another indicator. For example, a coordinate value of two points (points 512 and 513 in FIG. 5C) at which the C plane intersects with the mitral annulus may be estimated and a vector that is orthogonal to a vector connecting the two points may be calculated as the short axis vector after correction. Alternatively, a coordinate value of two points (not illustrated) which differ from the two points and through which a short axis is to pass may be estimated and a vector connecting the two estimated points may be calculated as the short axis vector after correction.

In addition, the short axis vector after correction may be calculated by image processing without using a learning model. For example, a vector in a direction of a maximum diameter of an annulus drawn on the C plane cross sectional image may be calculated and a vector that is orthogonal to the vector may be used as the short axis vector. In a similar manner, a configuration may be adopted in which even in estimation processing of a correction parameter using the A plane cross sectional image or the B plane cross sectional image, a feature point position necessary for correction is estimated by image processing such as template matching and correction is performed using the feature point position.

In the present embodiment, all three cross sections being the A plane, the B plane, and the C plane are considered calculation objects of a correction amount and when the cross sectional image acquiring unit 43 acquires two or more cross sectional images, an indicator estimated by the correction parameter estimating unit 45 differs for each cross sectional image. However, all three cross sections need not necessarily be considered calculation objects of a correction amount. For example, only one cross section may be considered an object or the two cross sections being the A plane and the B plane may be considered objects. In this case, with respect to a component of which a correction amount has not been calculated among the reference cross section parameter, an estimated value as the initial parameter can be used as the final parameter. Alternatively, correction amounts of two or more types of components may be calculated from one cross sectional image such as calculating correction amounts of both the center position and the long axis vector using the A plane cross sectional image.

In addition, while a case of using a two-dimensional cross sectional image in the estimation of the correction parameter in step S205 has been described as an example in the present embodiment, a three-dimensional image made up of a small number of slices may be used instead. In this case, based on the initial parameter, the cross sectional image acquiring unit 43 acquires cross sectional images in association with a plurality of sliced images corresponding to a prescribed sliced thickness in the three-dimensional image from the three-dimensional image. For example, an input to a CNN may be a three-dimensional image made up of a small number of slices (for example, two previous and two subsequent slices centered on an output cross sectional image) and an output may be an in-plane component correction amount in a similar manner to the processing described above. Accordingly, correction amount estimation in consideration of an image feature of a nearby three-dimensional space can be performed. Alternatively, a correction amount in the three-dimensional space may be estimated without limiting the output to in-plane components. In other words, for example, a correction amount of the three-dimensional coordinates of the center position is calculated from a three-dimensional image made up of slices in a periphery of the A plane cross sectional image. Accordingly, a correction amount calculation with higher accuracy can be performed.

(Step S206: Correction of Initial Parameter) In step S206, the correcting unit 46 corrects the initial parameter using the initial parameter estimated in step S203 and the correction amount estimated in step S205 and calculates the final parameter.

The correction of the initial parameter is performed according to the following procedure. First, a correction amount (a movement amount or a rotation amount) defined on the two-dimensional space of each cross section is converted to a movement amount or a rotation amount in a three-dimensional space. Next, the initial parameter of the reference cross section is corrected using the converted movement amount or rotation amount to calculate the final parameter. In other words, in the previous step S205, the correction amount independently estimated for each component for each cross sectional image is simultaneously reflected in the present step. When the center position or an axis vector after the correction is estimated instead of the correction amount in step S205, the center position or the axis vector of which an estimated value is converted into the three-dimensional space coordinate system that is a coordinate system of an input image is adopted as a value of the final parameter.

When a correction amount regarding a same component of the parameter is to be calculated in a plurality of cross sections in step S205, a selection or a combination of correction amounts is performed in the present step. Specifically, for example, when a movement amount in the A plane of the center position is estimated from the A plane cross section and a movement amount in the B plane of the center position and a rotation amount in the B plane of the long axis vector are estimated from the B plane cross section, two correction amounts being the movement amount in the A plane and the movement amount in the B plane are to exist regarding the center position. In this case, with respect to a movement amount in the up-down direction which is a common component of the two movement amounts, a value integrating both movement amounts (for example, a simple mean value or a weighted average value) is used. Accordingly, the two correction amounts can be reflected in a well-balanced manner. Otherwise, a reliability of correction parameter calculation processing may be evaluated and a correction amount to be adopted may be selected based on the reliability. For example, based on an intention of assuming that estimation of a correction amount has failed when the correction amount is equal to or larger than a prescribed value (for example, a movement amount of the center position of 10 mm), only correction results in which an absolute value of the movement amount is equal to or smaller than the prescribed value may be adopted and corrected. Accordingly, the risk that a correction result with low reliability which is far removed from the initial parameter is output can be reduced. Alternatively, a correction amount may be weighted on the basis of a certain criterion. For example, weighting may be applied on the basis of a contrast of a cross sectional image so that a correction amount according to a high-contrast cross sectional image is more emphasized. Accordingly, a correction amount that emphasizes a correction amount with higher reliability can be calculated.

(Step S207: Acquisition of Sectional Image) In step S207, the cross sectional image acquiring unit 43 segments a two-dimensional cross sectional image using the input three-dimensional image and the reference cross section parameter after correction estimated in step S206. The acquired two-dimensional cross sectional image is a cross sectional image in which the center position and each axis vector of a reference cross section are corrected as described above.

(Step S208: Display of Estimation Result) In step S208, the display processing unit 51 displays the cross sectional image obtained by cutting the input three-dimensional image using the final parameter in step S207 in an image display region of the display unit 36 in a display mode that enables the cross sectional image to be readily visible.

Instead of performing display based on the final parameter, display may be performed in a display mode that combines a "cross sectional image based on the initial parameter" acquired in step S204 and the "correction amount" calculated in step S205. For example, cross sectional images are generated on the basis of the initial parameter of the reference cross section. In addition, information that visualizes a movement amount of the center position or a rotation amount of a vector by an arrow or the like is superimposed on the cross sectional images. In this case, the correction processing of step S206 can be omitted. Furthermore, when the object is to perform an analysis or a measurement based on the reference cross section, the display processing in step S208 is not essential and a configuration may be adopted in which respective cross sectional images are generated from an input three-dimensional image using the final parameter obtained in step S206 and the cross sectional images are stored in a storage apparatus or output to the outside. Moreover, a configuration may be adopted in which the final parameter obtained in step S206 is output to the storage unit 34 or to the outside.

According to the present embodiment, in processing of calculating a reference cross section parameter from a three-dimensional image, an initial parameter estimated from a low-resolution three-dimensional image is corrected on the basis of a two-dimensional cross sectional image. Accordingly, highly accurate estimation can be performed without increasing a calculation amount.

### Modification 1 of First Embodiment

Hereinafter, modifications of the first embodiment will be described. While an example in which a processing object is a three-dimensional ultrasonic image capturing a mitral valve region of the heart has been shown in the first embodiment, the technique according to the present disclosure can also be implemented when using an image that captures a region of the heart other than the mitral valve, an image of an organ other than the heart, or images by other modalities.

Examples of applying the present invention to an image of a region other than the mitral valve region of the heart photographed by a modality other than an ultrasonic image include a case of detecting a prescribed structure such as the diencephalon or the corpus callosum from a brain MRI image. When detecting such structures, for example, an axial cross section that passes a position where the structures are visible can be used as a reference cross section. In this case, first, an initial value of the reference cross section (axial cross section) is calculated by CNN. Next, images of the axial cross section and a coronal cross section and a sagittal cross section which are respectively orthogonal to the axial cross section are acquired. Furthermore, point coordinates indicating positions of prescribed anatomical landmarks that represent a structure of the brain on the images are estimated. Finally, a parameter representing the axial cross section is corrected using the estimated values of the point coordinates of the landmarks.

As described above, according to the present modification, the technique according to the present disclosure can be applied to images of modalities other than a three-dimensional ultrasonic image and when a region other than the mitral valve of the heart is used as an object.

### Modification 2 of First Embodiment

Next, another modification of the first embodiment will be described. While "a relationship between an image and a parameter value" was learned using a CNN when estimating an initial value of a reference cross section parameter in step S203 in the first embodiment, the technique according to the present disclosure can also be implemented in methods other than learning the relationship between an image and a parameter.

An example of other methods is a method of discriminating "whether or not an input image is appropriate as a reference cross section" using a known discriminator such as Random Forests. In this case, in a learning stage, first, a three-dimensional image for learning is segmented on the basis of a large number of sectional parameters. At this point, an image segmented based on a parameter that is appropriate as a reference cross section (close to a reference cross section) is labeled a "reference cross section" and an image segmented based on a parameter that is not appropriate as a reference cross section (far from a reference cross section) is labeled "not a reference cross section". In this case, being close to or far from the reference cross section indicates a case where a difference in position and attitude from the reference cross section is smaller than or equal to or larger than a prescribed value (for example, 5.0 mm and 5.0 degrees). In addition, the labeled images are input to the Random Forests to train the discriminator. In an estimating state, a large number of random sectional parameters are generated, a large number of cross sectional images are segmented from the input three-dimensional image using the parameters, and "whether or not the cross sectional image is a reference cross section" is discriminated with respect to each cross sectional image using the discriminator. Furthermore, a mean value of parameters used when generating cross sectional images determined to be a "reference cross section" is output as an estimation result of the reference cross section parameter.

Alternatively, the initial parameter can also be estimated by applying an algorithm not based on learning data. For example, with respect to each cross sectional image (A plane, B plane, or C plane) of the reference cross section, a two-dimensional template representing a typical aspect may be prepared and template matching of searching for a position and an attitude which result in a good fit of the template among the input three-dimensional image may be performed. Furthermore, instead of having the image processing apparatus 10 automatically estimate the initial parameter, input of an initial value of the reference cross section parameter by the user may be accepted via the operating unit 35.

In this manner, according to the present modification, an initial value of the reference cross section parameter may be determined based on a wide variety of methods in addition to a CNN.

### Modification 3 of First Embodiment

In the first embodiment, a case where the position and the attitude of three cross sections (the A plane, the B plane, and the C plane) in an observation object coordinate system are known and a position and an attitude of each reference cross section are uniquely calculated from a common reference cross section parameter has been described as an example. However, the technique according to the present disclosure can also be applied to a case where each reference cross section is independently defined.

As an example, a case where the three cross sections being the A plane, the B plane, and the C plane do not share a center position and are not orthogonal to each other will be described as an example. While the three cross sections are expressed using nine parameters (three parameters of a position, three parameters of a long axis vector, and three parameters of a short axis vector) in the first embodiment, when the three cross sections are mutually independent, nine parameters are necessary to express each cross section. In other words, the reference cross section parameter according to the present modification is not a parameter that represents a position and an attitude of an observation object but a parameter that directly represents a position and an attitude of each reference cross section. Therefore, in step S203, a total of 27 parameters are estimated as initial parameters of the reference cross section. In addition, while a correction amount of an in-plane component of each cross section is calculated in step S205 in a similar manner to the first embodiment, when the respective cross sections are mutually independent, a correction amount calculated using a given cross section does not affect another cross section. In other words, a correction amount estimated for each cross section is independently applied to each cross section.

Note that each cross section may be completely independent as described above or, alternatively, with respect to a given cross section and another cross section, the cross sections may be configured so as to share a part of parameters and only another part of the parameters are independent such as the cross sections sharing a center position and a long axis and only an angle at which the planes intersect with each other being indeterminate. In such a case, only the indeterminate parameter is estimated as the initial parameter. In this case, the correction of a parameter of a given cross section is performed only when a correction amount calculated using the cross section affects a parameter that is shared with the other cross section.

Alternatively, in addition to cases where the three reference cross sections being the A plane, the B plane, and the C plane are used, the technique according to the present disclosure can be applied in a similar manner to the first embodiment to cases where there are one to two or four or more reference cross sections as long as the reference cross sections can be represented by a prescribed number of parameters.

### Second Embodiment

Next, a second embodiment will be described. It should be noted that, in the following description, components and processing similar to those of the first embodiment will be denoted by the same reference signs and detailed descriptions thereof will be omitted. A configuration of the image processing apparatus 10 according to the present embodiment is the configuration shown in FIG. 1.

In a similar manner to the first embodiment, the image processing apparatus 10 according to the second embodiment is an apparatus which receives a three-dimensional ultrasonic image obtained by photographing a heart region as an input and which estimates a reference cross section for observation. In the first embodiment, the correction amount estimated in step S205 is unconditionally reflected onto a final estimation result. However, as in the present embodiment, a determination of whether or not to reflect an estimated correction amount onto a final result may be made based on a certain criterion. Accordingly, when there are a plurality of indicators estimated by the correction parameter estimating unit 45, the correcting unit 46 may determine, for each indicator, whether or not to correct an initial parameter based on the indicator.

As will be described below, in the image processing apparatus 10 according to the present embodiment, processing by the correcting unit 46 and the display processing unit 51 differs from the processing in the first embodiment.

The correcting unit 46 calculates the final parameter by applying the correction amount of the reference cross section parameter estimated by the correction parameter estimating unit 45 onto the initial parameter estimated by the initial parameter estimating unit 42. In doing so, a determination result by the user with respect to whether or not to accept a correction result is acquired via the operating unit 35 to determine whether or not the correction amount of the reference cross section parameter is to be applied. When it is determined that the correction amount of the reference cross section parameter is to be applied, the correction amount of the reference cross section parameter is applied to the initial parameter.

The display processing unit 51 includes, in addition to the function of the display processing unit 51 described in the first embodiment, a function of displaying a cross sectional image based on the reference cross section parameter in a display mode that enables the user to determine whether or not to accept a correction result of the reference cross section parameter. Details of the processing by the correcting unit 46 and the display processing unit 51 will be described later in the description of step S406.

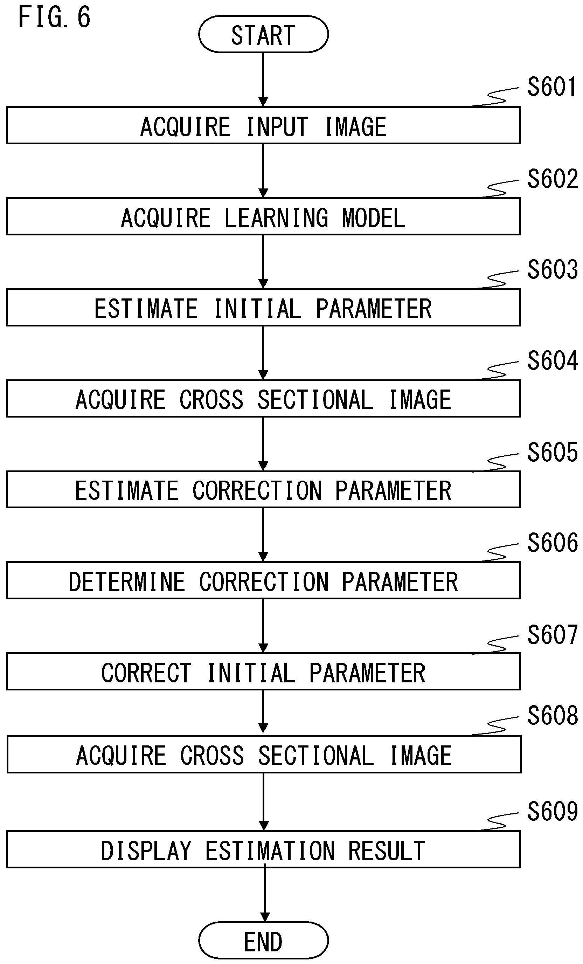

Next, an example of processing of the image processing apparatus 10 according to the second embodiment will be described with reference to the flow chart shown in FIG. 6. In this case, processing of steps S601 to S605 and steps S608 and S609 are the same as the processing of steps S201 to S205 and steps S207 and S208 in the flow chart (FIG. 2) according to the first embodiment. Hereinafter, only processing steps that differ from the first embodiment will be described.

(Step S606: Determination of Correction Parameter) In step S606, by controlling the operating unit 35 and the display processing unit 51, the correcting unit 46 determines, for each component of a reference cross section parameter, whether or not to adopt a correction amount of the reference cross section parameter estimated in step S605. Hereinafter, processing procedures will be specifically described.

First, the display processing unit 51 performs image display necessary for the user to determine a determination result. For example, a cross sectional image generated based on the reference cross section parameter prior to the correction (initial parameter) and a cross sectional image generated based on the reference cross section parameter onto which the correction amount is reflected are displayed side by side. By comparing the cross sectional image generated based on the initial parameter and the cross sectional image generated with the parameter onto which the correction amount is reflected, the user can determine whether or not the correction amount is appropriate.

A different display method from the above may be used as long as the user can determine an appropriateness of the correction amount. For example, an arrow or the like indicating a correction amount (movement amount) of the center position or a correction amount (rotation amount) of an axis vector may be superimposed and displayed on a cross sectional image generated based on an initial parameter prior to the correction. Alternatively, as shown in FIG. 5A to 5C, center positions or axis vectors before the correction and after the correction may be indicated by a dot or a straight line.

Secondly, the correcting unit 46 acquires a selection result of "whether or not to accept a correction result" by the user through the operating unit 35. At this point, the correcting unit 46 accepts the selection result of whether or not to accept a correction result for each component of the reference cross section parameter. In other words, the correcting unit 46 determines whether or not to accept a correction result with respect to each of the three components being the center position, the long axis vector, and the short axis vector.

In addition to any of the selection results regarding whether a correction result is accepted or not accepted, the correcting unit 46 may accept an input of a correction result by the user and correct the initial parameter based on the accepted correction result. For example, the user may operate the operating unit 35 while checking a cross sectional image after the correction and correct the center position and an attitude (specifically, a value of an axis vector) on the cross sectional image in an interactive manner. In this case, the correcting unit 46 adopts a correction instruction from the user as a new correction amount. In addition, an instruction of whether or not a correction result obtained in this manner is to be accepted is stored in the RAM 33 together with the correction amount. Alternatively, instead of making a selection as to whether or not a correction result is to be accepted, the user may always manually perform a correction based on the initial parameter.

Instead of the user manually determining whether or not a correction result is to be accepted, whether or not a correction result is to be accepted may be automatically determined on the basis of a prescribed criterion. For example, when the correction amount (in other words, a movement amount of the center position or a rotation amount of an axis vector) calculated in step S605 is larger than a prescribed value, the correcting unit 46 may determine that an estimation of the correction amount has failed and may determine not to accept the correction result. Alternatively, the correcting unit 46 may acquire a discriminator that quantitatively evaluates an "appropriateness (likelihood) of each cross sectional image" for each cross sectional image and may determine that an estimation of a correction amount has failed when a likelihood as a result of discrimination of a cross sectional image after the correction by the discriminator falls below a prescribed value. In addition, for example, the correcting unit 46 may display a message prompting the user to modify the correction amount on the display unit 36 when the correcting unit 46 determines that estimation of the correction amount has failed based on such an automatic determination result.

(Step S607: Correction of Initial Parameter) In step S607, using the initial parameter estimated in step S603 and the correction amount estimated in step S605, the correcting unit 46 corrects the initial parameter and calculates the final parameter. The processing in the present step is the same as the processing in step S206 according to the first embodiment with the exception of using a determination result of the correction amount in step S606. In other words, the correcting unit 46 only reflects a parameter of a component with respect to which a determination of "a correction result is to be accepted" is made in step S606 onto the correction of the initial parameter.

As described above, according to the present embodiment, using a determination by the user in the correction of the initial parameter enables estimation of a reference cross section parameter to be performed more flexibly and with higher quality.

### Third Embodiment

Next, a third embodiment will be described. It should be noted that, in the following description, components and processing similar to those of the first and second embodiments will be denoted by the same reference signs and detailed descriptions thereof will be omitted.

In a similar manner to the first and second embodiments, an image processing apparatus according to the third embodiment is an apparatus configured to estimate a reference cross section of an input three-dimensional image. A configuration of the image processing apparatus 10 according to the present embodiment is the configuration shown in FIG. 1.

In addition, the image processing apparatus 10 according to the present embodiment calculates an initial parameter of a reference cross section and calculates a correction amount using a cross sectional image generated based on the initial parameter in a similar manner to the first and second embodiments. In the first and second embodiments, correction amounts of three components being the center position, the long axis vector, and the short axis vector are estimated independently (in parallel) and the correction amounts are simultaneously reflected onto a final parameter. However, in the present embodiment, estimation of a correction amount of each component and reflection of the correction amount onto the reference cross section parameter are sequentially performed. In other words, in the image processing apparatus 10 according to the present embodiment, an order of components to be corrected is determined in advance. Information regarding the order of components to be corrected may be stored in the storage unit 34 or the like.

In addition, first, the correction parameter estimating unit 45 performs estimation of a correction amount with respect to a first component and the cross sectional image acquiring unit 43 acquires a cross sectional image (cross sectional image after correction) that is segmented based on the reference cross section parameter having been corrected based on the estimated first component. Furthermore, the correction parameter estimating unit 45 performs estimation of the correction amount with respect to a second component based on the cross sectional image after correction. In a similar manner, the cross sectional image acquiring unit 43 acquires a cross sectional image after correction that is corrected on the basis of the estimation result and the correction parameter estimating unit 45 performs estimation of the correction amount with respect to a third component based on the cross sectional image after correction.

The cross sectional image acquiring unit 43 generates a two-dimensional cross sectional image by cutting, based on the reference cross section parameter, an input three-dimensional image acquired by the image acquiring unit 61 in a similar manner to the first embodiment. Furthermore, in addition to generating a cross sectional image based on the initial parameter of the reference cross section estimated by the initial parameter estimating unit 42, the cross sectional image acquiring unit 43 also generates a cross sectional image based on the reference cross section parameter corrected by the correcting unit 46.

The correction parameter estimating unit 45 calculates a correction amount of a component corresponding to a type of each cross sectional image based on the two-dimensional cross sectional image generated by the cross sectional image acquiring unit 43 in a similar manner to the first embodiment. However, unlike the first embodiment, instead of simultaneously and independently performing calculations of all of the correction amounts of the A plane, the B plane, and the C plane, the correction parameter estimating unit 45 only performs estimation of the correction amount of a cross section that is a present object based on the order determined in advance. For example, when it is determined that "corrections are to be performed in an order of the A plane, the B plane, and the C plane", the correction parameter estimating unit 45 performs an estimation of a correction amount using a cross sectional image of the B plane after an estimation of a correction amount of the A plane and a reflection of the correction amount onto the reference cross section parameter are completed. Furthermore, the correction parameter estimating unit 45 performs an estimation of a correction amount using a cross sectional image of the C plane after an estimation of a correction amount of the B plane and a reflection of the correction amount onto the reference cross section parameter are completed. A specific order of processing will be described later.

The correcting unit 46 corrects the reference cross section parameter using the correction amount estimated by the correction parameter estimating unit 45 in a similar manner to the first embodiment. In the first embodiment, the correcting unit 46 corrects the reference cross section parameter using all of the correction amounts of the A plane, the B plane, and the C plane. On the other hand, in the present embodiment, the correcting unit 46 corrects the reference cross section parameter using only the correction amount of a component used as an estimation object by the correction parameter estimating unit 45.

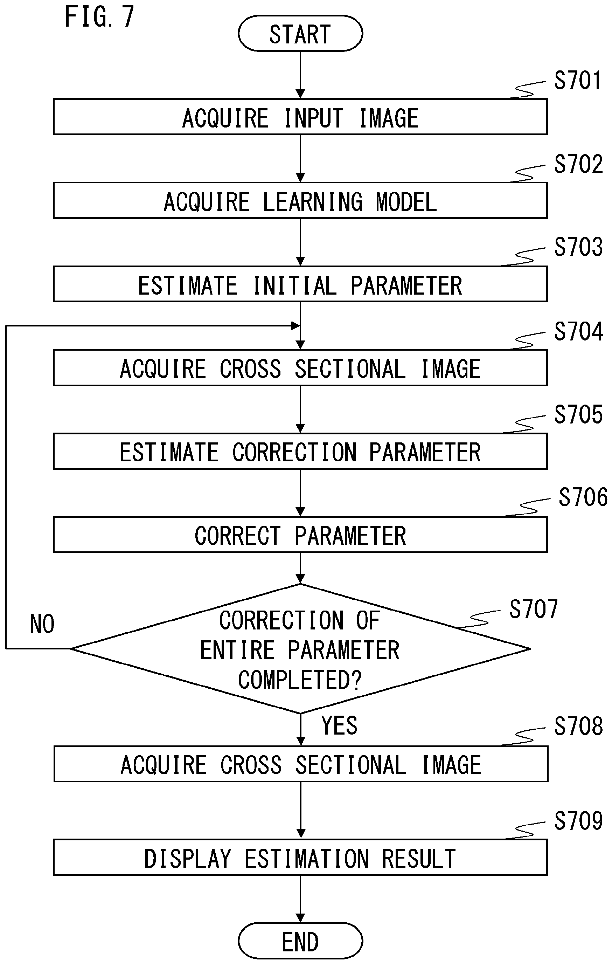

Next, an example of processing of the image processing apparatus 10 according to the present embodiment will be described with reference to the flow chart shown in FIG. 7. Since processing in S701 to S703 and S708 and S709 are respectively the same as the processing in S201 to S203 and S207 and S208 in the flow chart shown in FIG. 2, a detailed description will be omitted here.

(Step S704: Acquisition of Sectional Image) In step S704, the cross sectional image acquiring unit 43 generates a two-dimensional cross sectional image by cutting, based on the reference cross section parameter, an input three-dimensional image acquired by the image acquiring unit 41.

In step S204 in the first embodiment, a two-dimensional cross sectional image is always calculated on the basis of the initial parameter estimated in step S203. However, in the present embodiment, when processing returns to step S704 after advancing through steps S705 to S707 in a latter stage, in step S704, a two-dimensional cross sectional image is acquired on the basis of the reference cross section parameter corrected in step S706.

For example, when processing returns to step S704 after correction of the reference cross section parameter using the A plane is performed in step S706, the cross sectional image acquired in S704 is a cross sectional image of a component corresponding to the A plane or, in other words, a cross sectional image in a state where only the center position is corrected. In this case, in step S704, a cross sectional image is acquired by the cross sectional image acquiring unit 43 by adopting the center position after the correction as the center position but adopting initial parameters for the other components (long axis vector and short axis vector).

(Step S705: Estimation of Correction Parameter) In step S705, using the cross sectional image acquired in step S704, the correction parameter estimating unit 45 estimates a correction amount of a component of the reference cross section parameter corresponding to a type of the cross section.

Unlike in step S205 in the first embodiment, in step S705, estimation of a correction parameter with respect to a cross sectional image of a given type is performed according to an order determined in advance. For example, when an order of the A plane, the B plane, and the C plane has been determined as an order of correction and when performing step S705 in a state where correction of the A plane has been completed, an estimation of a parameter correction amount is performed with respect to the B plane cross sectional image in step S705. The estimation processing of a correction amount with respect to each cross sectional image is the same as the processing in step S205 according to the first embodiment.

(Step S706: Correction of Parameter) In step S706, the correcting unit 46 calculates the reference cross section parameter after correction using the correction amount of the parameter estimated in step S705.

In step S206 in the first embodiment, with an initial parameter as an input, all of the correction amounts of the center position, the long axis vector, and the short axis vector were simultaneously reflected onto the initial parameter. On the other hand, in the processing of step S705 according to the present embodiment, the initial parameter is corrected with respect to components of which a correction amount has been estimated among the parameter obtained by the processing in steps S704 to S707. In other words, an update is performed with respect to a part of the components of the reference cross section parameter. Note that the processing itself of correcting the reference cross section parameter using an estimated correction amount is the same as the processing according to the first embodiment.

(Step S707: Determination of Whether or not Correction of Entire Parameter has been Completed) In step S707, the correcting unit 46 determines whether or not the correction of all components of the reference cross section parameter that is a correction object has been completed. For example, when an order of the A plane, the B plane, and the C plane has been determined as an order of correction and when the correction of the respective cross sections has been completed, the correcting unit 46 assumes that the correction of the initial parameter has been completed (S707: YES) and advances processing to step S708. On the other hand, when there is a cross section of which correction has not been completed or, in other words, when a part of the components of the initial parameter remains as the reference cross section parameter, the correcting unit 46 assumes that correction has not been completed (S707: NO) and returns processing to step S704.

According to the present embodiment, based on a cross sectional image onto which a correction of a given component (for example, the center position) of the reference cross section parameter is reflected, a correction amount of a remaining component (for example, the long axis vector) is estimated. In other words, the image processing apparatus 10 according to the present embodiment sequentially executes correction of the reference cross section parameter. Therefore, according to the present embodiment, compared to a case where correction amounts of the respective components of the reference cross section parameter are independently estimated and simultaneously reflected, the possibility of an occurrence of a mismatch or an inconsistency among correction results of the respective components of the reference cross section parameter can be reduced. Accordingly, a reference cross section parameter with a higher quality can be calculated.

While a case where the reflection of correction amounts is performed in an order of the A plane, the B plane, and the C plane has been described as an example of the present embodiment, a different order may be adopted. In addition, a correction of any one cross section need not be performed. Alternatively, whether or not a correction amount is to be reflected may be determined for each cross section in combination with the processing described in the second embodiment. In this manner, the correction parameter estimating unit 45 is capable of estimating an indicator based on a part of a plurality of images that make up a cross sectional image and sequentially switching, in a prescribed order, which image among the plurality of images is to be used as a basis when estimating the indicator.

While this concludes the description of the present embodiment, the configurations and processing of the electronic device described above are not limited to the embodiments described above and various modifications can be made without losing identity with the technical concepts of the present invention. For example, an estimation accuracy of the reference cross section parameter can be enhanced by appropriately performing the processing according to the respective embodiments in combination with each other.

### Other Embodiments

The technique according to the present disclosure can take the form of an embodiment of, for example, a system, an apparatus, a method, a program, or a recording medium (a storage medium). Specifically, the technique according to the present disclosure may be applied to a system constituted by a plurality of devices (for example, a host computer, an interface device, an image capturing apparatus, and a web application) or to an apparatus constituted by a single device.

It is needless to say that the object of the present invention can be realized by the following. Specifically, a recording medium (or a storage medium) on which is recorded a program code (a computer program) of software that realizes functions of the embodiments described above is supplied to a system or an apparatus. It is needless to say that the storage medium is a computer-readable storage medium. In addition, a computer (or a CPU or an MPU) of the system or the apparatus reads and executes the program code stored in the recording medium. In this case, the program code itself having been read from the recording medium is to realize the functions of the embodiments described above and the recording medium on which the program code is recorded is to constitute the present invention.

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)^{™}), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An image processing apparatus comprising:
an image acquiring means configured to acquire a three-dimensional image of an object;
an initial parameter estimating means configured to reduce a resolution of the three-dimensional image and estimate an initial parameter in accordance with an initial value of an adjustable parameter to be utilized in obtaining a reference cross section;
a cross sectional image acquiring means configured to acquire one or more cross sectional images based on the three-dimensional image and the initial parameter; and
an indicator estimating means configured to estimate, an indicator to be contributed to correcting the initial parameter, wherein the estimated indicator is in association with a specified cross sectional image out of the one or more cross sectional images.

2. The image processing apparatus according to claim 1, wherein the cross sectional image acquiring means is configured to acquire a plurality of cross sectional images in association with a plurality of sliced volume image corresponding to a prescribed sliced thickness based on the three-dimensional image and the initial parameter.

3. The image processing apparatus according to claim 1 or 2, wherein a resolution of the cross sectional image acquired by the cross sectional image acquiring means is higher than a resolution of the three-dimensional image after reducing a resolution thereof.

4. The image processing apparatus according to any one of claims 1 to 3, wherein when the cross sectional image acquiring means is configured to acquire a plurality of cross sectional images, a plurality of indicators estimated by the indicator estimating means in association with the plurality of cross sectional images are different from each other.

5. The image processing apparatus according to any one of claims 1 to 4, wherein the initial parameter estimating means is configured to estimate the initial parameter based on the three-dimensional image reduced in the resolution.

6. The image processing apparatus according to any one of claims 1 to 5, further comprising:
a learning model acquiring means configured to acquire a learning model, wherein
the learning model is at least any one of a learning model for initial parameter estimation created by learning processing using information including a set of a three-dimensional image for learning and a parameter for obtaining the reference cross section in the three-dimensional image for learning and a learning model for indicator estimation created by learning processing using information including a set of a cross sectional image for learning and an indicator that contributes toward correcting a reference cross section parameter in the cross sectional image for learning, and
at least any one of the initial parameter estimating means and the indicator estimating means performs the estimation based on the learning model.

7. The image processing apparatus according to any one of claims 1 to 6, wherein the initial parameter is information related to a position or an attitude of the reference cross section in the three-dimensional image.

8. The image processing apparatus according to claim 7, wherein the indicator estimated by the indicator estimating means is an indicator that contributes toward correction in an image plane of the cross sectional image of the initial parameter.

9. The image processing apparatus according to any one of claims 1 to 8, wherein the indicator estimated by the indicator estimating means is a correction amount for moving a reference cross section that is obtained by the initial parameter.

10. The image processing apparatus according to claim 9, wherein the correction amount is at least one of an amount of displacement for moving a position of the reference cross section obtained by the initial parameter to a target position of the reference cross section and a rotation amount for rotating an attitude of the reference cross section obtained by the initial parameter to a target attitude of the reference cross section.

11. The image processing apparatus according to any one of claims 1 to 8, wherein
the indicator estimated by the indicator estimating means is a parameter for obtaining the reference cross section, and
the indicator estimating means estimates a parameter for obtaining the reference cross section based on the initial parameter.

12. The image processing apparatus according to claim 11, wherein the parameter for obtaining the reference cross section is at least one of an estimated value of a target position of the reference cross section and an estimated value of an axis vector to be a target of the reference cross section.

13. The image processing apparatus according to claim 11, wherein the indicator estimating means performs an estimation of positions of a plurality of prescribed landmarks on a cross sectional image and estimates a parameter for obtaining the reference cross section based on a result of the estimation of the positions.

14. The image processing apparatus according to any one of claims 1 to 8, further comprising:
a correcting means configured to correct the initial parameter based on the indicator estimated by the indicator estimating means.

15. The image processing apparatus according to claim 14, wherein the correcting means determines whether or not to perform a correction of the initial parameter based on the indicator estimated by the indicator estimating means, and performs the correction of the initial parameter based on a result of the determination.

16. The image processing apparatus according to claim 15, wherein when there are a plurality of indicators estimated by the indicator estimating means, the correcting means determines, for each indicator, whether or not to correct the initial parameter based on the indicator.

17. The image processing apparatus according to claim 16, further comprising:
a display processing means configured to perform processing of displaying the three-dimensional image, the cross sectional image, and the indicators estimated by the indicator estimating means, wherein
the correcting means is configured to acquire a selection result by a user with respect to an indicator among the indicators estimated by the indicator estimating means based on which a correction of the initial parameter is to be performed, and performs the correction of the initial parameter based on the selection result.

18. The image processing apparatus according to claim 16, further comprising:
a display processing means configured to perform processing of displaying the three-dimensional image, the cross sectional image, and the indicators estimated by the indicator estimating means, wherein
the correcting means corrects, based on an instruction from a user, the indicators estimated by the indicator estimating means and performs a correction of the initial parameter based on the corrected indicators.

19. The image processing apparatus according to claim 14, wherein
the cross sectional image is made up of a plurality of images of which positions and attitudes among the images have a prescribed relationship,
the cross sectional image acquiring means is configured to acquire a cross sectional image after correction from the three-dimensional image based on a sectional parameter corrected by the correcting means, and
the indicator estimating means further performs estimation of the indicators based on the cross sectional image after correction.

20. The image processing apparatus according to claim 19, wherein
the indicator estimating means estimates the indicators based on a part of the plurality of images that make up the cross sectional image, and
the indicator estimating means sequentially switches, in a prescribed order, which an image among the plurality of images is to be used as a basis when estimating the indicators.

21. An image processing method, comprising:
an image acquiring step of acquiring a three-dimensional image of an object;
an estimating step of reducing a resolution of the three-dimensional image and estimating an initial parameter in accordance with an initial value of an adjustable parameter to be utilized in obtaining a reference cross section;
a cross sectional image acquiring step of acquiring one or more cross sectional images based on the three-dimensional image and the initial parameter; and
an indicator estimating step of estimating an indicator to be contributes to correcting the initial parameter, wherein the estimated indicator is in association with a specified cross sectional image out of the one or more cross sectional images.

22. A program for causing a computer to execute each any one of a plurality of steps in the image processing method according to claim 21.
